# EUROPÄISCHE PATENTSCHRIFT

(11) **EP 2 435 442 B1**
(45) Veröffentlichungstag und Bekanntmachung des Hinweises auf die Patenterteilung: **13.01.2016**
(21) Anmeldenummer: 10723985.7
(22) Anmeldetag: 25.05.2010
(51) Int. Cl.: C07D 501/04, C07D 501/18, C07D 501/56

(54) **VERFAHREN ZUR HERSTELLUNG VON CEFTOBIPROL MEDOCARIL**
METHOD FOR PRODUCING CEFTOBIPROL MEDOCARIL
PROCÉDÉ DE FABRICATION DE CEFTOBIPROL MEDOCARIL

(30) Priorität: 25.05.2009 EP 09161028
(43) Veröffentlichungstag der Anmeldung: 04.04.2012
(73) Patentinhaber: Sandoz AG, 4056 Basel (CH)
(72) Erfinder: KREMMINGER, Peter, A-6250 Kundl (AT); LUDESCHER, Johannes, A-6250 Kundl (AT); STURM, Hubert, A-6250 Kundl (AT)
(74) Vertreter: Herzog, Fiesser & Partner Patentanwälte PartG mbB
(86) Internationale Anmeldenummer: PCT/EP2010/057105
(87) Internationale Veröffentlichungsnummer: WO 2010/136423

(56) Entgegenhaltungen:
- EP-A- 0 849 269
- EP-B- 1 087 980
- WO-A-95/29182
- WO-A1-02/14332

## Beschreibung

Die vorliegende Erfindung betrifft ein Verfahren zur Herstellung von organischen Verbindungen, insbesondere von Natrium (6R,7R)-7-[(Z)-2-(5-Amino-[1,2,4]thiadiazol-3-yl)-2-hydroxyimino-acetylamino]-8-oxo-3-[(E)-(R)-1'-(5-methyl-2-oxo-[1,3]-dioxol-4-ylmethoxycarbonyl)-2-oxo-[1,3']bipyrrolidinyl-3-ylidenemethyl]-5-thia-1-aza-bicyclo[4.2.0]oct-2-ene-2 carboxylat (Ceftobiprol Medocaril), bzw. Verbindungen der allgemeinen Formel (1) und der allgemeinen Formel (2), die Verbindungen selbst sowie Intermediate in der erfindungsgemäßen Herstellung. Ceftobiprol Medocaril ist ein parenterales Cephalosporin mit hervorragenden antibakteriellen Eigenschaften. Einen Überblick gibt beispielsweise Current Opinion in Pharmacology 2006, 6, 480-485.

Verfahren zur Herstellung von Ceftobiprol Medocaril sind an sich bekannt. Den aus dem Stand der Technik bekannten Verfahren ist gemeinsam, dass ausgehend von 7-Aminocephalosporansäure eine Vielzahl an Zwischenstufen hergestellt, isoliert und aufgereinigt werden müssen, um Ceftobiprol Medocaril der allgemeinen Formel (1) in ausreichender Reinheit zu erhalten.

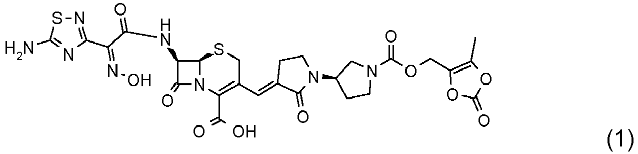

Die Verbindung der allgemeinen Formel (1) ist an sich bekannt und beispielsweise in WO 99/65920 beschrieben. Sie ist verwendbar für die Behandlung und zur Prophylaxe von bakteriellen Infektionskrankheiten, insbesonders von Infektionskrankheiten, die durch Methicillin resistente Staphylococcus Aureus Stämme hervorgerufen werden.

WO 99/65920 beschreibt als letzten Schritt des Herstellprozesses von Ceftobiprol Medocaril eine Umsetzung, wobei bei einer Verbindung der allgemeinen Formel (2) die Medocaril-Prodrug Einheit eingeführt wird.

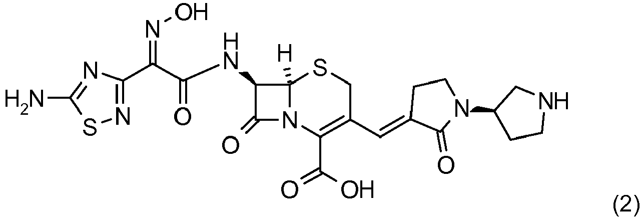

Die Verbindung der allgemeinen Formel (2) ist ebenfalls an sich bekannt und wurde beispielsweise in EP 0 849 269 A1 beschrieben. Die Herstellung der Verbindung der allgemeinen Formel (2) erfolgt gemäß EP 0 849 269 A1 ausgehend von (2R,6R,7R)-tert. Butoxycabonylamino-3-formyl-8-oxo-5-thia-1-azabicyclo[4.2.0]oct-3-ene-2-carboxylic acid benzhydrylester durch Wittig-Reaktion mit (1'-Allyloxycarbonyl-2-oxo-[1,3']bipyrrolidinyl-3-yl)-triphenylphosphonium bromid. Das dabei entstehende Δ2-Reaktionsprodukt wird durch Sulfoxidierung und anschließender Reduktion zum gewünschten Δ3-Isomeren rückisomerisiert und danach mit Trifluoressigsäure vom Benzhydrylester entschützt. Die Acylierung in Position 7 erfolgt durch Reaktion mit (Z)-(5-Amino-[1,2,4]-thiadiazol-3-yl)-trityloxyiminothioessigsäure S-benzothiazol-2-yl ester. Die Verbindung der allgemeinen Formel (2) wird im Anschluss durch Abspaltung der Schutzgruppen erhalten.

In EP 1 067 131 A1 wird die Bildung des Ylids in Toluol oder einer Mischung aus Toluol und Dichlormethan durch Zugabe von Alkali tert. Butylat in Tetrahydrofuran beschrieben, wodurch die Base als Lösung zugegeben werden kann. Die Umsetzung des Ylids mit dem entsprechenden Aldehyd ist bei einer Reaktionstemperatur von - 70°C beschrieben.

EP 0 841 339 A1 betrifft Cephalosporin Derivate sowie Verfahren zu deren Herstellung. WO 95/29182 offenbart ebenfalls Zwischenprodukte für die Herstellung von Cephalosporinen.

WO 01/90111 beschreibt eine weitere Herstellung von Ceftobiprol Medocaril in mehreren Stufen ausgehend von Desacetyl-7-aminocephalosporansäure durch Acylierung mit (Z)-(5-Amino-[1,2,4]-thiadiazol-3-yl)-trityloxyiminothioessigsäure S-benzothiazol-2-yl ester in N,N-Dimethylformamid, gefolgt von in situ Veresterung mit Diphenyldiazomethan in Dichlormethan zum entsprechenden Benzhydrylester, der durch Zugabe von Hexan gefällt und isoliert wird. Dieses Produkt wird im nächsten Schritt mit TEMPO/ NaOCl in Dichlormethan/Wasser oder mit Braunstein in Tetrahydrofuran/Dichlormethan zum entsprechenden Aldehyd oxidiert. Der nächste Reaktionschritt beinhaltet die Wittig-Reaktion zum 3-Vinylsubstituierten Derivat, bei der in Dichlormethan/Toluol/Tetrahydrofuran bei -78°C umgesetzt wird. Das Rohprodukt wird mit Ethanol ausgerührt und aus Dichlormethan/tert. Butylmethylether umkristallisiert bzw. chromatographisch gereinigt. Gemäß dem in WO 01/90111 offenbarten Verfahren wird die Wittigreaktion bei tiefen Temperaturen von -80 bis - 70°C in einem komplexen Lösungsmittelgemisch aus Dichlormethan, Toluol und Tetrahydrofuran durchgeführt. Dies führt bei der Durchführung der Reaktion im Produktionsmaßstab zu erheblichen Nachteilen, da eine Regenerierung der Prozesslösungsmittel erschwert ist.

Nachteilig an den aus dem Stand der Technik bekannten Synthesen ist, dass die Verbindung der allgemeinen Formel (2) bzw. der allgemeinen Formel (1) über einen vielstufigen Prozeß hergestellt wird, der komplexe Syntheseschritte beinhaltet und schlechte Gesamtausbeuten liefert. Darüber hinaus sind aufwendige Schutzgruppenoperationen nötig.

Es wurde nun überraschenderweise gefunden, dass Verbindungen der allgemeinen Formel (1) bzw. der allgemeinen Formel (2) über ein Verfahren hergestellt werden können, das mit wenigen Stufen auch im industriellen Maßstab durchführbar ist.

Soweit nicht explizit ausgeführt betreffen die folgenden Ausführungen im Rahmen der vorliegenden Erfindung jeweils die genannten Verbindungen selbst sowie deren pharmazeutisch verträgliche Salze.

Die vorliegende Erfindung betrifft demgemäß ein Verfahren zur Herstellung einer Verbindung der allgemeinen Formel (1) 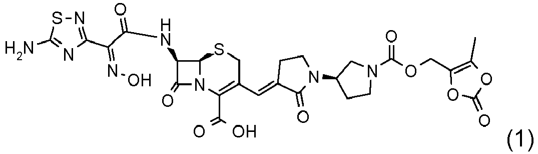 mindestens umfassend die folgenden Schritte (a), (b), (c) und (d):
(a) Umsetzung einer Verbindung der allgemeinen Formel (3) 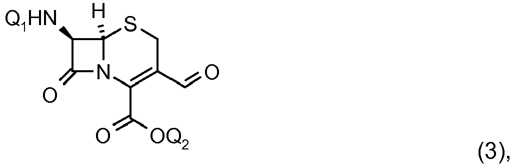 wobei Q₁ und Q₂ unabhängig voneinander für eine Silylgruppe steht oder wobei Q₁ für ein Wasserstoffatom und Q₂ für eine Silylgruppe steht,
   mit einer Verbindung der allgemeinen Formel (4) 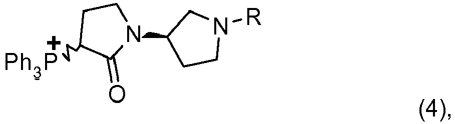 wobei R für eine BOC-Gruppe steht, zu einer Verbindung der allgemeinen Formel (5) 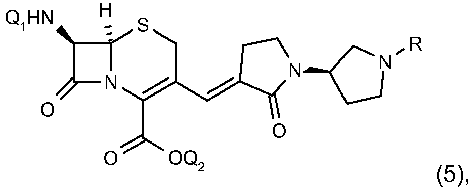 wobei Q₁, Q₂ und R wie oben definiert sind;
(b) Umsetzung der Verbindung der allgemeinen Formel (5) mit einer Verbindung der allgemeinen Formel (6) 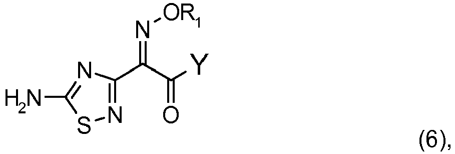 wobei R₁ für eine Hydroxy-Schutzgruppe und Y für eine aktivierende Funktionalität steht,
   gegebenenfalls nach Entfernung der Schutzgruppe, falls Q₁ für eine Silylgruppe steht,
   zu einer Verbindung der allgemeinen Formel (7) 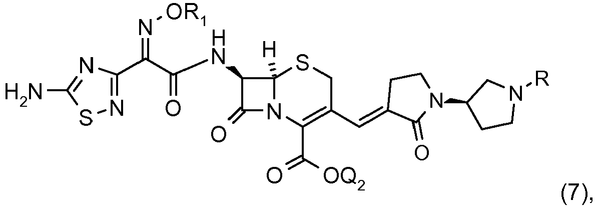 wobei R₁, Q₂ und R wie oben definiert sind;
(c) Umsetzung der Verbindung der allgemeinen Formel (7) zu der Verbindung der allgemeinen Formel (2), nach Entfernung der Schutzgruppe, 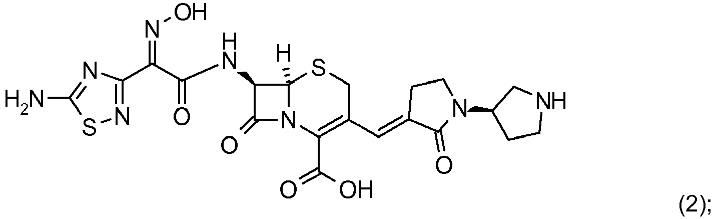 und
(d) Umsetzung der Verbindung der allgemeinen Formel (2) zu einer Verbindung der allgemeinen Formel (1).

Darüber hinaus betrifft die vorliegende Erfindung ein Verfahren zur Herstellung einer Verbindung der allgemeinen Formel (2) 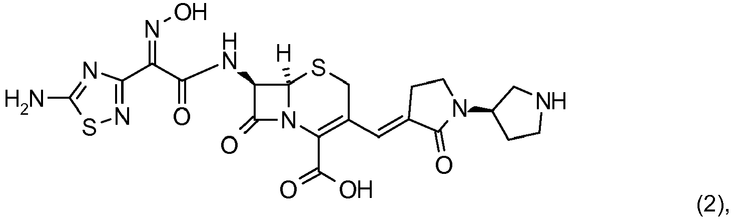 mindestens umfassend die folgenden Schritte (a), (b) und (c):
(a) Umsetzung einer Verbindung der allgemeinen Formel (3) 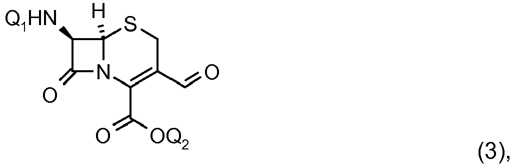 wobei Q₁ und Q₂ unabhängig voneinander für eine Silylgruppe steht oder wobei Q1 für ein Wasserstoffatom und Q2 für eine Silylgruppe steht,
   mit einer Verbindung der allgemeinen Formel (4) 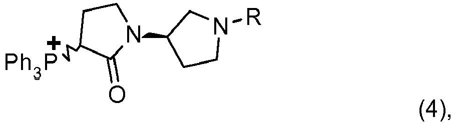 wobei R für eine BOC-Gruppe steht,
   zu einer Verbindung der allgemeinen Formel (5) 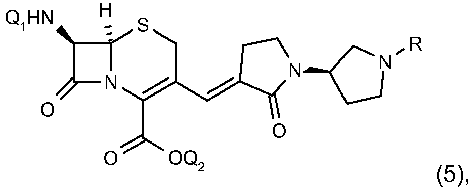 wobei Q₁, Q₂ und R wie oben definiert sind;
(b) Umsetzung der Verbindung der allgemeinen Formel (5) mit einer Verbindung der allgemeinen Formel (6) 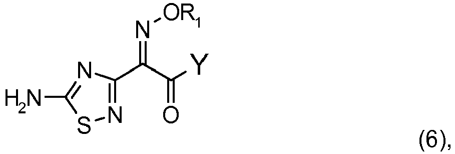 wobei R₁ für eine Hydroxy-Schutzgruppe und Y für eine aktivierende Funktionalität steht,
   gegebenenfalls nach Entfernung der Schutzgruppe, falls Q₁ für eine Silylgruppe steht,
   zu einer Verbindung der allgemeinen Formel (7) 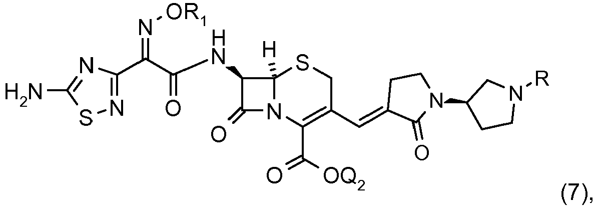 wobei R₁, Q₂ und R wie oben definiert sind; und
(c) Umsetzung der Verbindung der allgemeinen Formel (7) zu der Verbindung der allgemeinen Formel (2), gegebenenfalls nach Entfernung der Schutzgruppe.

Die erfindungsgemäßen Verfahren umfassen mindestens die Schritte (a), (b) und gegebenenfalls (c). Die erfindungsgemäßen Verfahren können darüber hinaus auch weitere Schritte umfassen, beispielsweise Schutzgruppenoperationen. Dabei ist es erfindungsgemäß möglich, dass diese weiteren Schritten vor oder nach den Schritten (a), (b) und (c) erfolgen. Ebenso ist es jedoch möglich, dass die weiteren Schritte zwischen den Schritten (a) und (b) oder zwischen den Schritten (b) und (c) erfolgen.

Das erfindungsgemäße Verfahren ermöglicht die Herstellung der Verbindung der allgemeine Formel (1) oder der allgemeinen Formel (2) in einfacher Weise und in hoher Reinheit.

Das erfindungsgemäße Verfahren umfasst Schritt (a), d.h. die Umsetzung einer Verbindung der allgemeinen Formel (3) 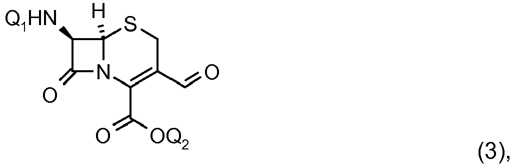 wobei Q1 und Q2 unabhängig voneinander für eine Silylgruppe stehen oder wobei Q1 für ein Wasserstoffatom und Q2 für eine Silylgruppe steht,
mit einer Verbindung der allgemeinen Formel (4) 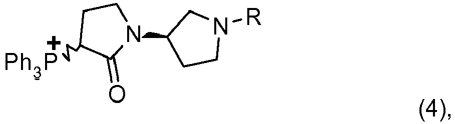 wobei R für eine Boc-Gruppe steht,
zu einer Verbindung der allgemeinen Formel (5) 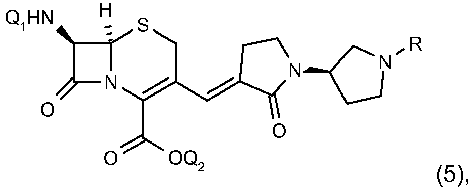 wobei Q₁, Q₂ und R wie oben definiert sind.

Erfindungsgemäß kann die Umsetzung gemäß Schritt (a) in jeder dem Fachmann bekannten Weise durchgeführt werden. Dabei wird die Verbindung der allgemeinen Formel (3) mit dem Phosphoniumsalz der allgemeinen Formel (4) umgesetzt. Die Umsetzung erfolgt erfindungsgemäß in Gegenwart einer Base unter Bildung der Verbindung der allgemeinen Formel (5).

Geeignete Reaktionsbedingungen und Lösungsmittelsysteme sind beispielsweise in WO 95/29182 auf Seite 26, zweiter Absatz bis Seite 27, einschließlich vorletzter Absatz beschrieben, sowie in Beispiel 31 auf Seite 42 in WO 95/29182.

Die Umsetzung gemäß Schritt (a) kann erfindungsgemäß beispielsweise in Gegenwart eines Silylierungsmittels und/oder eines Epoxids durchgeführt werden, insbesondere in Gegenwart von BSA und/oder Propylenoxid.

Daher betrifft die vorliegende Erfindung auch ein Verfahren zur Herstellung einer Verbindung der allgemeinen Formel (1) oder ein Verfahren zur Herstellung einer Verbindung der allgemeinen Formel (2) wie oben beschrieben, wobei Schritt (a) in Gegenwart eines Silylierungsmittels und eines Epoxids durchgeführt wird.

Gemäß bevorzugter Ausführungsformen betrifft die vorliegende Erfindung auch ein Verfahren zur Herstellung einer Verbindung der allgemeinen Formel (1) oder ein Verfahren zur Herstellung einer Verbindung der allgemeinen Formel (2) wie oben beschrieben, wobei es sich bei dem Silylierungsmittel um BSA handelt oder wobei es sich bei dem Epoxid um Propylenoxid handelt.

Die erfindungsgemäßen Verfahren umfassen darüber hinaus einen Schritt (b), d.h. die Umsetzung der Verbindung der allgemeinen Formel (5) mit einer Verbindung der allgemeinen Formel (6) 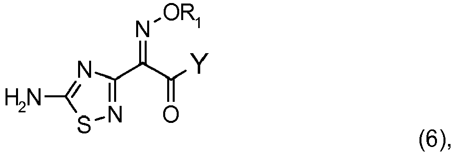 wobei R₁ für eine Hydroxy-Schutzgruppe und Y für eine aktivierende Funktionalität steht,
gegebenenfalls nach Entfernung der Schutzgruppe, falls Q₁ für eine Silylgruppe steht,
zu einer Verbindung der allgemeinen Formel (7) 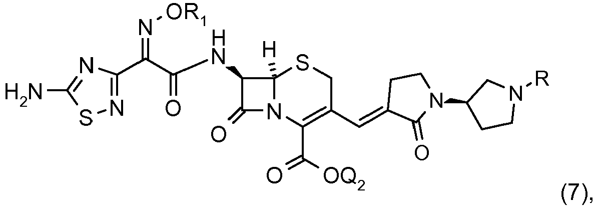 wobei R₁, Q₂ und R wie oben definiert sind.

Gemäß Schritt (b) des erfindungsgemäßen Verfahrens wird die Verbindung der allgemeinen Formel (5) mit der Verbindung der allgemeinen Formel (6) acyliert.

Erfindungsgemäß ist Y eine aktivierende Funktionalität wie beispielsweise ein Halogenid, wobei geeignete Halogenide beispielsweise in J. Antibiotics 37:557 - 571, 1984 offenbart werden, ein gemischtes Anhydrid, wobei geeignete gemischte Anhydride beispielsweise in Yakugaku Zasshi 110 (9) 658-664, 1990 offenbart werden, oder eine Gruppe ausgewählt aus den Gruppen (s), (t) und (u):

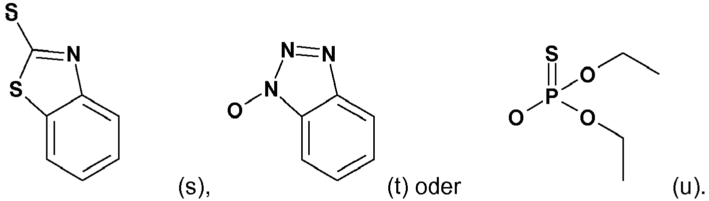

Dabei kann die Umsetzung gemäß Schritt (b) des erfindungsgemäßen Verfahrens grundsätzlich auf jede dem Fachmann bekannte geeignete Art durchgeführt werden.

Geeignete Reaktionsbedingungen und Lösungsmittelsysteme sind beispielsweise in EP 37380 A2 von Seite 8, Zeile 16 bis Seite 9, Zeile 5 beschrieben.

Erfindungsgemäß ist es bevorzugt, dass für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

Dabei kann im Rahmen der vorliegenden Erfindung Schritt (b) ohne Zwischenisolierung bzw. im Eintopfverfahren durchgeführt werden.

Gemäß einer bevorzugten Ausführungsform betrifft die vorliegende Erfindung daher auch ein Verfahren zur Herstellung einer Verbindung der allgemeinen Formel (1) oder ein Verfahren zur Herstellung einer Verbindung der allgemeinen Formel (2) wie oben beschrieben, wobei für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

Das erfindungsgemäße Verfahren kann neben den Schritten (a) und (b) auch noch einen Schritt (c) umfassen. Gemäß Schritt (c) erfolgt die Umsetzung der Verbindung der allgemeinen Formel (7) zu der Verbindung der allgemeinen Formel (2), nach Entfernung der Schutzgruppe.

Geeignete Reaktionsbedingungen und Lösungsmittelsysteme sind zum Beispiel in WO01/90111 auf Seite 11, Zeile 3 bis 27 beschrieben, insbesonders in Beispiel 5 und 6 von WO01/90111.

Erfindungsgemäß ist es bevorzugt, dass Schritt (c) ohne Zwischenisolierung bzw. im Eintopfverfahren durchgeführt wird. Gemäß einer weiteren Ausführungsform betrifft die vorliegende Erfindung daher auch ein Verfahren zur Herstellung einer Verbindung der allgemeinen Formel (1) oder ein Verfahren zur Herstellung einer Verbindung der allgemeinen Formel (2) wie oben beschrieben, wobei Schritt (c) ohne Zwischenisolierung des aus Schritt (b) erhaltenen Intermediats bzw. im Eintopfverfahren durchgeführt wird.

Es wurde überraschenderweise gefunden, dass mittels dieses erfindungsgemäßen Verfahrens eine Reduktion der Anzahl der isolierten Zwischenstufen bei der Herstellung von Verbindung der allgemeinen Formel (1) bzw. der allgemeinen Formel (2), insbesondere von Ceftobiprol Medocaril, erhalten werden kann, was letztlich zu höheren Gesamtausbeuten und in Folge zu günstigeren Herstellkosten führt.

Das erfindungsgemäßen Verfahren zur Herstellung der Verbindung der allgemeinen Formel (1) umfasst vorzugsweise neben den Schritten (a), (b) und (c) noch einen Schritt (d), wobei der Schritt (d) nach den Schritten (a), (b) und (c) erfolgt. Erfindungsgemäß ist es jedoch möglich, dass weitere Schritte, beispielsweise Schutzgruppenoperationen, zwischen den Schritten (c) und (d) erfolgen. Gemäß Schritt (d) erfolgt eine Umsetzung der Verbindung der allgemeinen Formel (2) zu einer Verbindung der allgemeinen Formel (1).

Dabei kann die Umsetzung gemäß Schritt (d) des erfindungsgemäßen Verfahrens grundsätzlich auf jede dem Fachmann bekannte geeignete Art durchgeführt werden.

Geeignete Reaktionsbedingungen und Lösungsmittelsysteme sind beispielsweise in WO99/65920 in Besispiel 1.1 auf den Seiten 9 und 10 beschrieben.

Die gemäß dem erfindungsgemäßen Verfahren erhaltenen Verbindungen zeichnen sich unter anderem durch eine hohe Reinheit aus.

Die vorliegende Beschreibung offenbart auch eine Verbindung der allgemeinen Formel (1) 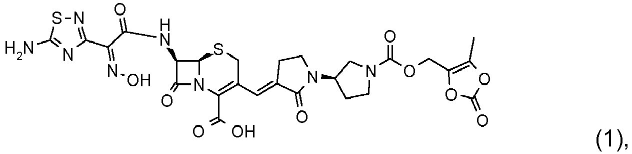 wobei die Verbindung erhältlich ist nach einem Verfahren wie oben beschrieben.

Die vorliegende Beschreibung offenbart weiter eine Verbindung der allgemeinen Formel (2) 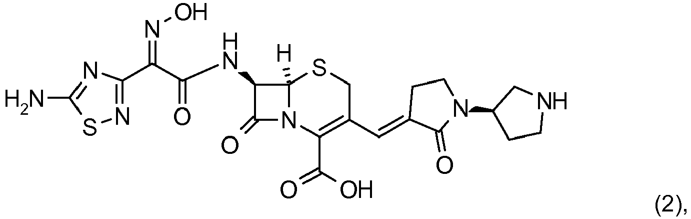 sowie Salze davon, insbesondere Salze mit starken organischen Säuren, wie das Trifluoressigsäuresalz oder das Tosylat oder Mesylat, wobei die Verbindung erhältlich ist nach einem Verfahren wie oben oder in den Beispielen beschrieben.

Die Verbindung der allgemeinen Formel (2) wird gemäß dem erfindungsgemäßen Verfahren in hoher Reinheit, beispielsweise >99 Flächenprozent erhalten, beispielsweise in einer Reinheit von 99,5 Flächen-%, bestimmt mittels HPLC, sodass die Verbindung der allgemeinen Formel (1) daraus ohne weiteren Aufreinigungsschritt in pharmazeutisch einsetzbarer Qualität erhältlich ist.

Die vorliegende Erfindung betrifft auch neue Intermediate der Synthese. So betrifft die vorliegende Erfindung gemäß einem weiteren Aspekt eine Verbindung der allgemeinen Formel (5) 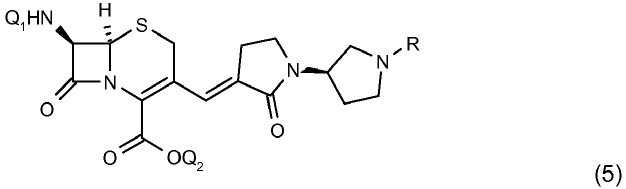 sowie Salze davon, insbesondere organische Aminsalze, wie z.B. das Dicyclohexylaminsalz aus Beispiel 3, wobei Q₁ und Q₂ unabhängig voneinander für eine Silylgruppe steht und R für eine Boc-Gruppe steht.

Demgemäß betrifft die vorliegende Erfindung gemäß einer weiteren Ausführungsform eine Verbindung der allgemeinen Formel (5) wie oben beschrieben, wobei Q₁ für eine Trimethylsilylgruppe und R für eine BOC-Gruppe steht.

Dieses BOC-geschützte Intermediat kristallisiert und fällt dann in sehr reiner Form an, beispielsweise 99% Flächenprozent HPLC oder reiner. Eine weiterer wesentlicher Vorteil der Verwendung des BOC-geschützten Intermediates bei der Herstellung von Ceftobiprol medocaril liegt darin, dass nach dem offengelegten Verfahren die eingesetzten Schutzgruppen BOC- und Triphenylmethyl- im Schritt (c) gleichzeitig abgespalten werden können, während in EP 0849 269 beschriebene Intermediate ALLOC-geschützt sind, sodass die Schutzgruppen in separaten Schritten abgespalten werden müssen. Die vorliegende Erfindung bezieht sich daher auch auf die Verwendung dieses BOC-geschützten Intermediates, also einer Verbindung der allgemeinen Formel (5) wie oben beschrieben, wobei Q₁ für eine Trimethylsilylgruppe und R für eine BOC-Gruppe steht, bei der Herstellung von Ceftobiprol medocaril.

Die vorliegende Erfindung betrifft auch Verbindungen der allgemeinen Formel (5) oder der allgemeinen Formel (7) erhältlich durch eine Umsetzung gemäß einem der Schritte (a) oder (b) wie oben beschrieben.

Die Verbindung der allgemeinen Formel (5) ist eine wesentliche Zwischenstufe des erfindungsgemäßen Verfahrens und ermöglicht die einfache Synthesesequenz des erfindungsgemäßen Verfahrens.

Die vorliegende Erfindung betrifft daher auch die Verwendung einer Verbindung der allgemeinen Formel (5) wie oben beschrieben zur Herstellung einer Verbindung der allgemeinen Formel (1) oder der allgemeinen Formel (2). Darüber hinaus betrifft die vorliegende Erfindung auch die Verwendung einer Verbindung der allgemeinen Formel (5) wie oben beschrieben zur Herstellung einer Verbindung der allgemeinen Formel (1).

Im Folgenden wird die vorliegende Erfindung anhand von Beispielen näher erläutert.

### 1. Referenzbeispiel: (6R,7R)-7-Amino-3[E-(R)-1'-(5-tert.butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure

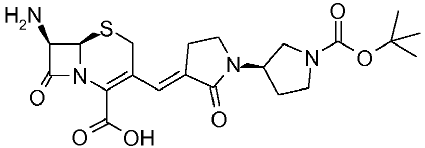

5 , 1 4 g 7-Amino-3-formyl-ceph-3-em-4-carboxylat wurden in 27,8 ml Bis(trimethylsilyl)acetamid und 50ml Propylenoxid gelöst. Anschließend wurden bei 1°C 16,8g (1 R/S,3'R)-(1'-tert.Butyloxycarbonyl-2-oxo-[1,3']bipyrrolidinyl-3-yl)-triphenylphosphonium Bromid (EP1067131, WO02/14332) langsam portionsweise zudosiert. Es wurde so lange bei 1°C nachgerührt, bis das Ausgangsmaterial abreagiert hat und danach wurde der kristalline Niederschlag unter Stickstoffatmosphäre abfiltriert und mit 50ml Cyclohexan/Bis(tirmethylsilyl)acetamid 99,5/0,5 gewaschen. Nach dem Trocknen unter Vakuum wurde das gewünschte Produkt in silylierter Form erhalten.

Das Material wurde in 100ml Dichlormethan gelöst und bei 0°C mit 50ml 3%-iger NaHCO₃ Lösung versetzt. Die Phasen wurden getrennt, die organische Phase mit 30ml Wasser gewaschen und die vereinigten Wasserphasen nach Aktivkohlebehandlung mit 3%-iger H₃PO₄ auf pH 3,5 gestellt. Der kristalline Niederschlag wurde filtriert, mit Wasser gewaschen und im Vakuum getrocknet.
Auswaage: 6,09g
¹H-nmr(DMSO-*d₆*) δ 1.39(s,9H), 2.00(m, 2H), 2.8-3.2(m, 2H), 3.2-3.5(m,6H), 3.84(ABq, 2H, J= 18.2Hz), 4.57(m,1H), 4.82(d,1H, J=5.1Hz), 5.01 (d,1 H, J=5.1Hz), 7.21 (m,1 H)
¹³C-nmr(DMSO-*d₆*) δ 24.63, 26.11, 28.09, 28.89, 41.54, 44.94, 45.31, 47.98, 48.34, 51.27, 52.00, 58.98, 63.76, 79.95, 121.95, 126.19, 126.28, 129.90, 134.21, 154.97, 164.36, 169.05, 169.13
MS- ESI negative mode: 927.2(2M-H, 100%, 463.1(M-H, 25%)
H₂O Gehalt: 2,2%
IR (golden gate, cm⁻¹): 2978, 1793, 1682, 1551, 1397, 1363, 1330

### 2. Beispiel: (6R,7R)-7-Trimethylsilylamino-3[E-(R)-1'-(5-tert.butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure trimethylsilylester

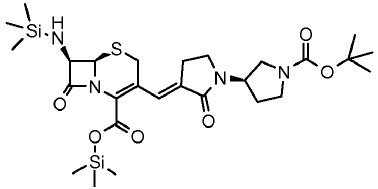

10,28 g 7-Amino-3-formyl-ceph-3-em-4-carboxylat wurden in 55,6 ml Bis(trimethylsilyl)acetamid und 100ml Propylenoxid gelöst. Anschließend wurden bei 0°C 33,6g (1R/S, 3'R)-(1'-tert. Butyloxycarbonyl-2-oxo-[1,3']bipyrrolidinyl-3-yl)-triphenylphosphonium Bromid (EP1067131, WO02/14332) langsam portionsweise über 22h zudosiert. Es wurde so lange bei 1°C nachgerührt, bis das Ausgangsmaterial abreagiert hat und danach wurde die Reaktionsmischung auf - 20°C abgekühlt. Der kristalline Niederschlag wurde unter Stickstoffatmosphäre abfiltriert und portionsweise mit 180ml Cyclohexan/Bis(trimethylsilyl)acetamid 99,5/0,5 gewaschen. Nach dem Trocknen unter Vakuum wurde das bissilylierte Produkt erhalten.
Auswaage: 16,2g
¹H-nmr(CDCl₃) δ 0.04,0.10,0.12(3s, 9H), 0,34(s, 9H), 1,43 (s, 9H), 1.74 (br s, 1 H), 1.9-2.2 (m, 2H), 2.8-3.0 (m,2H), 3.2-3.7 (m,8H), 4.7-4.95 (m, 3H), 7.43 (m, 1 H)

### 3. Beispiel: Dicyclohexylammonium (6R,7R)-7-Amino-3[E-(R)-1'-(5-tert. butyloxycarbonyl)-2-oxo-[1,3']bipyrrolidinyl-3-ylidenemethyl]-8-oxo-5-thia-1-aza-bicyclo[4.2.0]oct-2-ene-2-carboxylat

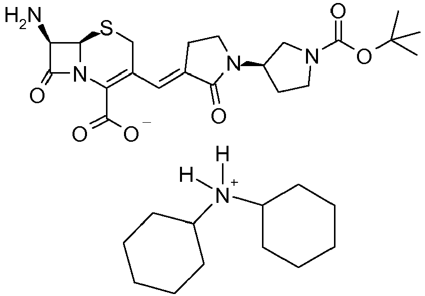

1,0g (6R,7R)-7-Trimethylsilylamino-3[E- (R)-1'-(5-tert. butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure trimethylsilylester wurden in 10ml Dichlormethan gelöst und mit einer Lösung von 300mg Dicyclohexylamin in 1ml EtOH und 10ml Ethylacetat versetzt. Der Niederschlag wurde abfiltriert, mit Ethylacetat gewaschen und im Vakuum getrocknet.
Auswaage: 0,9g
¹H-nmr(D₂O/DMSO-*d₆*) δ 0.9-1.3(m, 10H), 1.30(s,9H), 1.4-2.18m,12H), 2.7-3.5(m,10H), 3.64(ABq, J= 17.2Hz, 2H), 4.5 (m,1H)*, 4.58 (d,1H, J=5.1Hz); 4.88 (d,1 H, J=5.1 Hz), 7.07 (s,1 H)
* tlw. von D2O-Signal überlagert
MS- ESI negative mode: 927.2(2M-H, 100%), 463.1 (M-H, 25%)
IR (golden gate, cm ⁻¹): 2932, 2856, 1754, 1692, 1671, 1630, 1569, 1394, 1329

### 4. Beispiel: (6R,7R)-7-[(Z)-2-(5-Amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2 carbonsäure Trifluoracetat

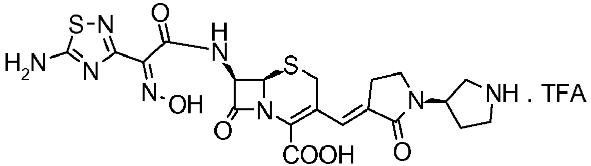

### 4.1 Variante A:

3,0g (6R,7R)-7-Amino-3[E-(R)-1'-(5-tert.butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure in silylierter Form wurden bei 0° in 150ml Dichlormethan gelöst. Anschließend wurden 600µl DMF/Wasser 5/1 und 1,8ml Bis(trimethylsilyl)acetamid zugegeben und 2,29g 2-Trityloxyimino-2-(5-amino-1,2,4-thiadiazol-3-yl)-essigsäure chlorid Hydrochorid (J. Antibiotics 37:557 - 571, 1984) wurden portionsweise zudosiert. Nach 3h bei 0° wurde die Mischung auf 30ml MeOH/120ml Wasser gegossen und die Methylenchlorid-Phase abgetrennt. Die organische Phase wurde auf 66g eingeengt und mit 25ml Trifluoressigsäure versetzt. Nach 10 Minuten wurden 1,5ml Triethylsilan und 10ml Wasser zugegeben und die Mischung wurde auf -15°C abgekühlt. Die organische Phase wurde abgetrennt und noch einmal mit 6ml Trifluoressigsäure/Wasser 1/1 gewaschen. Die vereinigten wäßrigen Phasen wurden mit Wasser auf 150ml verdünnt und über eine Adsorber-Harz Säule mit XAD-1600 filtriert. Nach dem Auswaschen der Säule mit Wasser wurde mit Wasser/Acetonitril 85/15 eluiert. Die produkthältigen Fraktionen wurde im Vakuum eingeengt und bei 0° zur Nachkristallisation stehen gelassen. Das kristalline Produkt wurde abfiltriert, mit Wasser gewaschen und im Vakuum getrocknet.
Auswaage: 2,66g

### 4.2 Variante B (Referenzbeispiel):

7,4g (6R,7R)-7-Amino-3[E-(R)-1'-(5-tert.butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure wurden bei 0° in 781 ml Dichlormethan unter Zugabe von 6,7ml Triethylamin gelöst. Anschließend wurden 8,65g 2-Trityloxyimino-2-(5-amino-1,2,4-thiadiazol-3-yl)-essigsäure chlorid Hydrochlorid portionsweise zudosiert. Nachdem das Ausgangsmaterial abreagiert war, wurde die Mischung auf 500ml Wasser gegossen und die Methylenchlorid-Phase abgetrennt. Die organische Phase wurde über Na₂SO₄ getrocknet, im Vakuum eingeengt.

Der Rückstand wurde in 148ml ml Dichlormethan gelöst und bei Raumtemperatur mit 4,5ml Triethylsilan und 74ml Trifluoressigsäure versetzt. Nach 30 Minuten wurden 222ml Dichlormethan und 222ml Wasser zugegeben und die Mischung wurde auf -20°C abgekühlt. Die organische Phase wurde abgetrennt und noch einmal mit einer Mischung aus 37ml Trifluoressigsäure und 148ml Wasser gewaschen. Die vereinigten wäßrigen Phasen wurden mit Wasser auf 364ml verdünnt, über ein Adsorberharz filtriert und mit Acetonitril /Wasser 15/85 eluiert. Das Filtrat am Rotavapor auf 35g eingeengt, filtriert und mit Wasser gewaschen. Nach dem Trocknen im Vakuum erhielt man 4,5g Auswaage.
¹H-nmr(DMSO-*d₆*) δ 1.9-2.2(m,2H), 2.8-3.5(m, 8H), 3.85(Abq, 2H; J=18.3Hz), 4.63(m,1 H), 5.16(d, 2H, J=4.9Hz), 5.85(dd, 1 H, J1= 4.9Hz, J2=8.4Hz), 7.23(s, 1 H), 8.06(s, 2H), 9.08 (br. s, 2H), 9.49(d, 2H, J=8.4Hz), 11.95 (s, 1 H)

### 5. Beispiel: (6R,7R)-7-[(Z)-2-(5-Amino-[1,2,4]thiadiazol-3-yl)-2-hydroxyimino-acetylaminol- 8-oxo-3-[(E)-(R)-2-oxo-[1,3']bipyrrolidinyl-3-ylidenemethyl]-5-thia-1-aza-bicyclo[4.2.0]oct-2-ene-2 carbonsäure

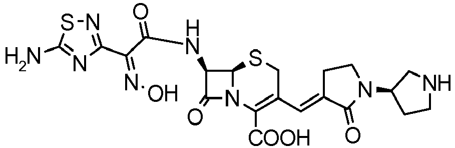

6,0g (6R,7R)-7-Amino-3[E-(R)-1'-(5-tert.butyloxycarbonyl)-2-oxo-[1,3']bipyrrolidinyl-3-ylidenemethyl]-8-oxo-5-thia-1-aza-bicyclo[4.2.0]oct-2-ene-2-carbonsäure in silylierter Form wurden bei 0° in 300ml Dichlormethan gelöst. Anschließend wurden 1200µl DMF/Wasser 5/1 und 8,1 ml Bis(tirmethylsilyl)acetamid zugegeben sowie 5,3g 2-Trityloxyimino-2-(5-amino-1,2,4-thiadiazol-3-yl)-essigsäure chlorid Hydrochorid (J. Antibiotics 37:557 - 571, 1984) portionsweise zudosiert. Danach wurde wurde die Mischung auf 60ml MeOH/240ml Wasser gegossen und die Methylenchlorid-Phase abgetrennt. Die organische Phase wurde auf 48g eingeengt und mit 1,5ml Triethylsilan versetzt. Nach Zugabe von 50ml Trifluoressigsäure wurde 60 min bei Raumtemperatur gerührt, 20 ml Wasser zugegeben und die Mischung wurde auf -15°C abgekühlt. Die organische Phase wurde abgetrennt und noch einmal mit 20ml Trifluoressigsäure/Wasser 1/1 gewaschen. Die vereinigten wäßrigen Phasen wurden mit Wasser auf 500ml verdünnt und mit 2,0g Aktivkohle behandelt. Nach der Filtration wurde die Lösung im Vakuum eingeengt.

Der Rückstand wurde mit Wasser auf 50ml verdünnt und mit gesättigter NaHCO₃-Lösung auf pH 6,9 eingestellt. Es wurde 2h bei 0°C nachgerührt, filtriert und der Niederschlag mit Wasser gewaschen.
Auswaage: 4,5g
¹H-nmr(DMSO-*d₆*/CF₃COOD) δ 1.9-2.3(m,2H), 2.8-3.5(m,8H), 3.85(ABq, 2H, 18.7Hz), 4.61(m,1H), 5.16(d, 1H,J=4.8Hz), 5.86(dd, 1H,J1=4.8Hz, J2=8.4Hz), 7.24(s,1 H), 8.05(br s, 2H), 8.93(s, 2H), 9.50(d,1 H,J=8.4Hz), 11.96(s, 1 H)
MS- ESI negative mode: 533.2(M-H, 10%)

### 6. Beispiel: Ceftobiprol Medocaril Na-Salz

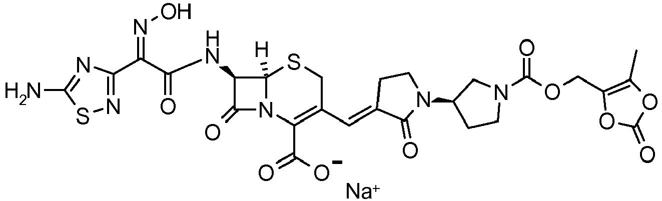

0,53g (6R,7R)-7-[(Z)-2-(5-Amino-[1,2,4]thiadiazol-3-yl)-2-hydroxyimino-acetylamino]-8-oxo-3-[(E)-(R)-2-oxo-[1,3']bipyrrolidinyl-3-ylidenemethyl]-5-thia-1-aza-bicyclo[4.2.0]oct-2-ene-2 carbonsäure wurden in 5ml Dimethylsulfoxid gelöst und mit 0,27g Kohlensäure-(5-methyl-2-oxo-[1,3]dioxol-4-ylmethyl)-4-nitrophenylester versetzt und bei Raumtemperatur gerührt. Zur Fällung wurde eine Lösung von Natrium Ethylhexanoat in 30ml Aceton zugegeben. Der Niederschlag wurde filtriert und mit Aceton gewaschen.
Auswaage: 0,6g
¹H-nmr(DMSO-*d₆*) δ 1.9-2.05(m, 2H), 2.10(s,3H), 2.7-3.1(m,2H), 3.1-3.6(m,6H), 3.64(q, 2H; J=17.1Hz), 4.56(m,1 H), 4.87(s,2H), 4.98(d,1 H,J=4.9Hz), 5.65(dd,1H,J1=4.9Hz, J2=8.4Hz), 7.34(s,1 H), 8.02(s,2H), 9.36(d,1H,J=8.4Hz)
MS- ESI negative mode: 689.0(M-H, 100%)

## Patentansprüche

1. Verfahren zur Herstellung einer Verbindung der allgemeinen Formel (1) 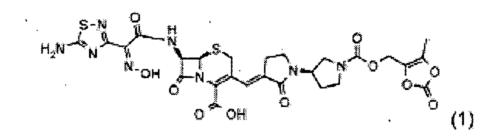 mindestens umfassend die folgenden Schritte (a), (b), (c) und (d):
(a) Umsetzung einer Verbindung der allgemeinen Formel (3) 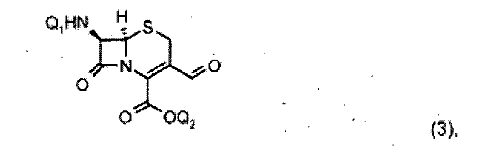 wobei Q₁ und Q₂ unabhängig voneinander für eine Silylgruppe steht oder
wobei Q₁ für ein Wasserstoffatom und Q₂ für eine Silylgruppe steht,
mit einer Verbindung der allgemeinen Formel (4) 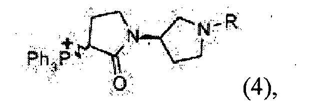 wobei R für eine BOC-Gruppe steht,
zu einer Verbindung der allgemeinen Formel (5) 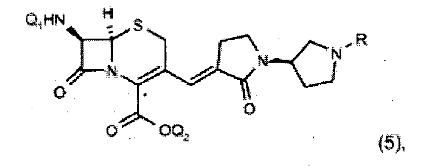 wobei Q₁, Q₂ und R wie oben definiert sind;
(b) Umsetzung der Verbindung der allgemeinen Formel (5) mit einer Verbindung der allgemeinen Formel (6) 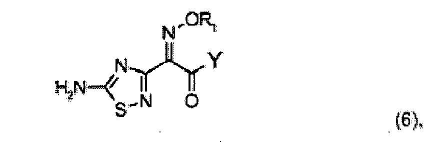 wobei R₁ für eine Hydroxy-Schutzgruppe und Y für eine aktivierende Funktionalität steht,
gegebenenfalls nach Entfernung der Schutzgruppe, falls Q₁ für eine Silylgruppe steht,
zu einer Verbindung der allgemeinen Formel (7) 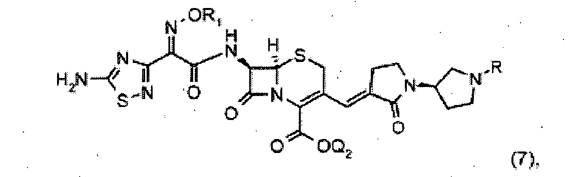 wobei R₁, Q₂ und R wie oben definiert sind;
(c) Umsetzung der Verbindung der allgemeinen Formel (7) zu der Verbindung der allgemeinen Formel (2), nach Entfernung der Schutzgruppe, 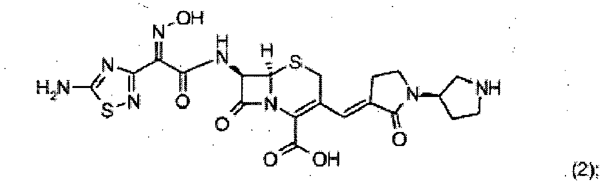 und
(d) Umsetzung der Verbindung der allgemeinen Formel (2) zu einer Verbindung der allgemeinen Formel (1).

2. Verfahren zur Herstellung einer Verbindung der allgemeinen Formel (2) 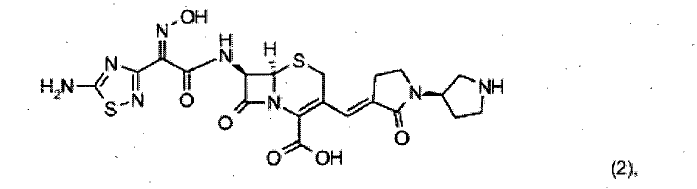 mindestens umfassend die folgenden Schritte (a), (b) und (c):
(a) Umsetzung einer Verbindung der allgemeinen Formel (3) 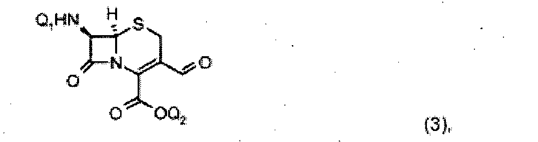 wobei Q₁ und Q₂ unabhängig voneinander für eine Silylgruppe steht oder
wobei Q₁ für ein Wasserstoffatom und Q₂ für eine Silygruppe steht,
mit einer Verbindung der allgemeinen Formel (4) 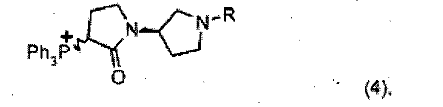 wobei R für eine BOC-Gruppe steht, zu einer Verbindung der allgemeinen Formel (5) 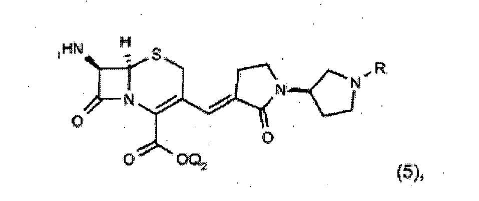 wobei Q₁, Q₂ und R wie oben definiert sind;
(b) Umsetzung der Verbindung der allgemeinen Formel (5) mit einer Verbindung der allgemeinen Formel (6) 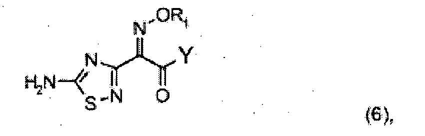 wobei R₁ für eine Hydroxy-Schutzgruppe und Y für eine aktivierende Funktionalität steht,
gegebenenfalls nach Entfernung der Schutzgruppe, falls Q₁ für eine Silylgruppe steht,
zu einer Verbindung der allgemeinen Formel (7) 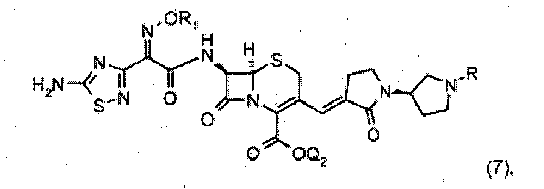 wobei R₁, Q₂ und R wie oben definiert sind;
und
(c) Umsetzung der Verbindung der allgemeinen Formel (7) zu der Verbindung der allgemeinen Formel (2), nach Entfernung der Schutzgruppe.

3. Verfahren nach Anspruch 1 oder 2, wobei Schritt (a) in Gegenwart eines Silylierungsmittels und eines Epoxids durchgeführt wird.

4. Verfahren nach Anspruch 3, wobei es sich bei dem Silylierungsmittel um BSA handelt.

5. Verfahren nach Anspruch 3 oder 4, wobei es sich bei dem Epoxid um Propylenoxid handelt.

6. Verfahren nach einem der Ansprüche 1 bis 5, wobei für die Umsetzung gemäß Schritt (b) die Verbindung der allgemeinen Formel (5) durch Silylierung oder Salzbildung gelöst wird, und nach erfolgter Acylierung mit der Verbindung der allgemeinen Formel (6) die Schutzgruppen in einem Schritt abgespalten werden.

7. Verfahren nach einem der Ansprüche 1 bis 6, wobei die Schritte (b) und (c) ohne Zwischenisolierung bzw. im Eintopfverfahren durchgeführt werden können.

8. Verbindung der allgemeinen Formel (5) oder (7) 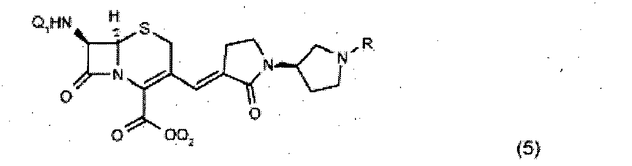 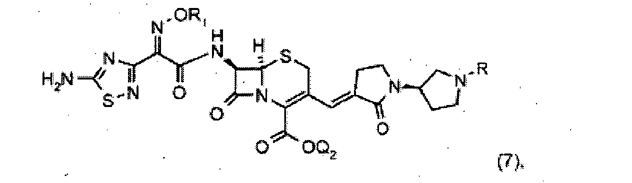 wobei R₁, Q₁, Q₂ und R gemäß Anspruch 1 definiert sind.

9. Verbindung der allgemeinen Formel (5) gemäß Anspruch 8, 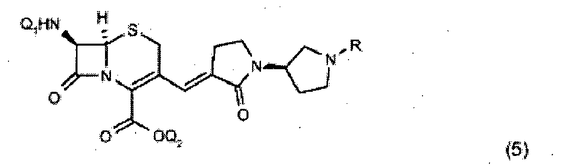 wobei Q₁ für eine Trimethylsilylgruppe, R für eine BOC-Gruppe und Q₂ für eine Silylgruppe steht.

10. Verwendung einer Verbindung gemäß Anspruch 9 zur Herstellung einer Verbindung der allgemeinen Formel (1)

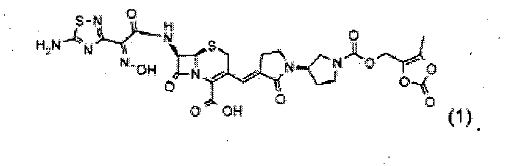

## Claims

1. Method for the production of a compound of the general formula (1) 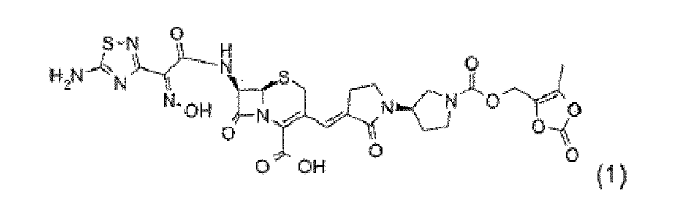 comprising at least the following steps (a), (b), (c) and (d):
(a) reaction of a compound of the general formula (3) 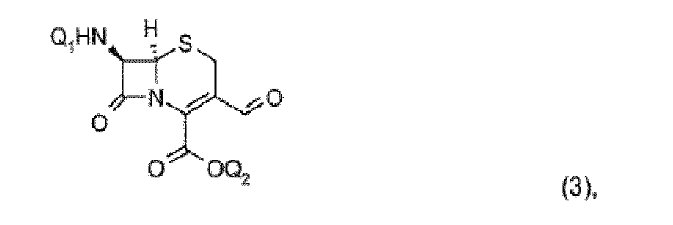 wherein Q₁ and Q₂ independently of each other represent a silyl group, or wherein Q₁ represents a hydrogen atom and Q₂ represents a silyl group,
with a compound of the general formula (4) 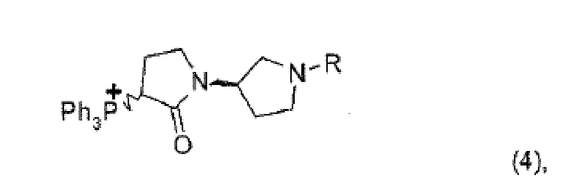 wherein R represents a BOC group,
to give a compound of the general formula (5) 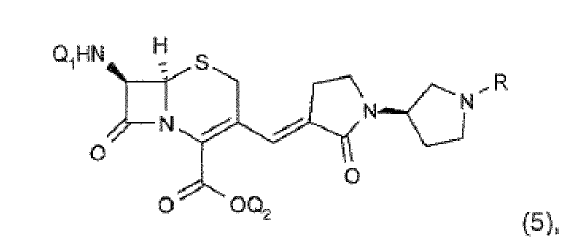 wherein Q₁, Q₂ and R are as defined above;
(b) reaction of the compound of the general formula (5) with a compound of the general formula (6) 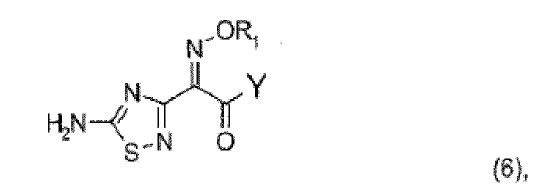 wherein R₁ represents a hydroxy-protective group and Y represents an activating functionality,
where appropriate after removal of the protective group, if Q₁ represents a silyl group,
to give a compound of the general formula (7) 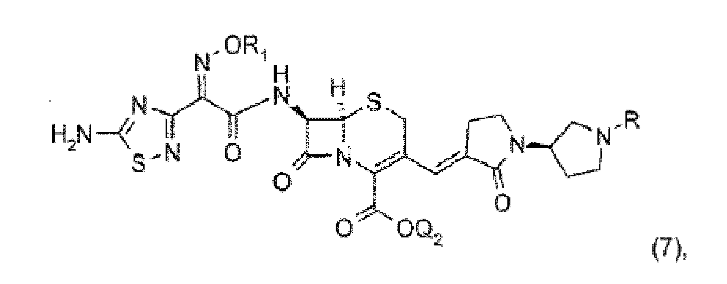 wherein R₁, Q₂ and R are as defined above;
(c) conversion of the compound of the general formula (7) into the compound of the general formula (2), after removal of the protective group, 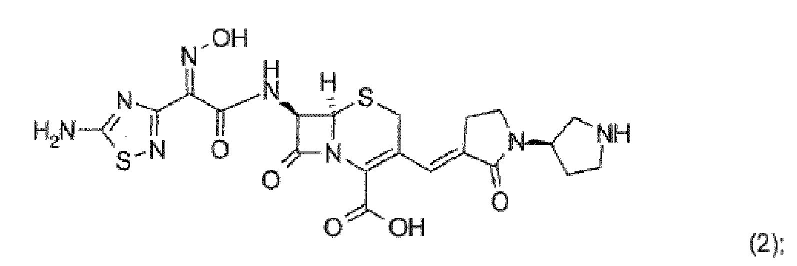 and
(d) conversion of the compound of the general formula (2) into a compound of the general formula (1).

2. Method for the production of a compound of the general formula (2) 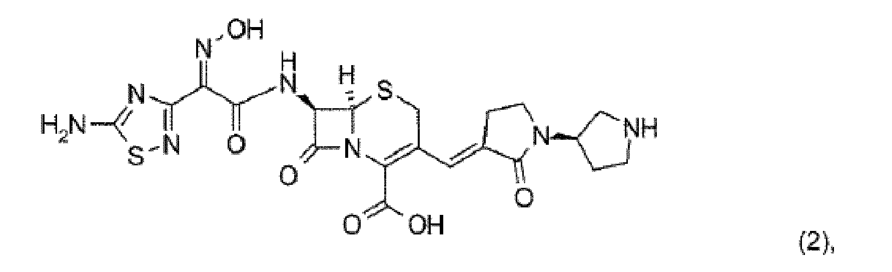 comprising at least the following steps (a), (b) and (c):
(a) reaction of a compound of the general formula (3) 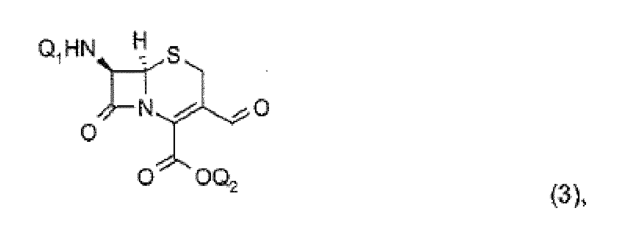 wherein Q₁ and Q₂ independently of each other represent a silyl group, or wherein Q₁ represents a hydrogen atom and Q₂ represents a silyl group,
with a compound of the general formula (4) 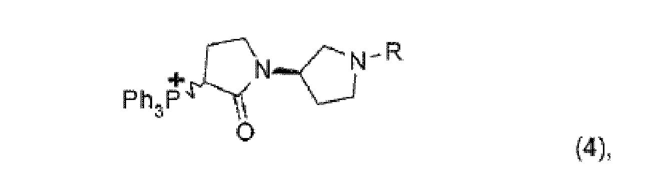 wherein R represents a BOC group,
to give a compound of the general formula (5) 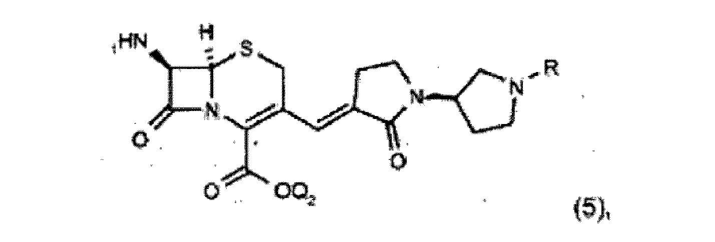 wherein Q₁, Q₂ and R are as defined above;
(b) reaction of the compound of the general formula (5) with a compound of the general formula (6) 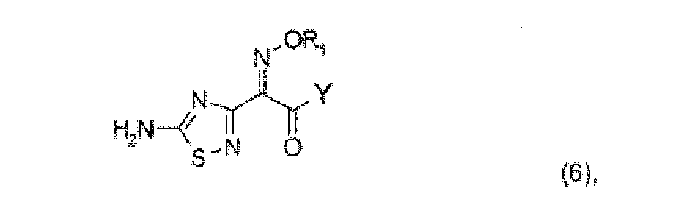 wherein R₁ represents a hydroxy-protective group and Y represents an activating functionality,
where appropriate after removal of the protective group, if Q₁ represents a silyl group,
to give a compound of the general formula (7) 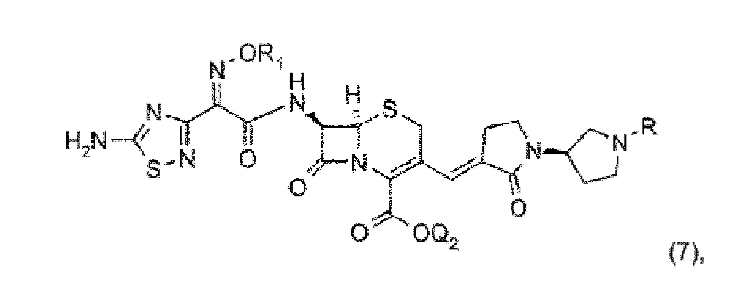 wherein R₁, Q₂ and R are as defined above; and
(c) conversion of the compound of the general formula (7) into the compound of the general formula (2), after removal of the protective group.

3. Method according to Claim 1 or 2, wherein step (a) is carried out in the presence of a silylating agent and an epoxide.

4. Method according to Claim 3, wherein the silylating agent is BSA.

5. Method according to Claim 3 or 4, wherein the epoxide is propylene oxide.

6. Method according to one of Claims 1 to 5, wherein for the reaction according to step (b) the compound of the general formula (5) is dissolved by silylation or salt formation, and when the acylation with the compound of the general formula (6) has been carried out, the protective groups are split off in one step.

7. Method according to one of Claims 1 to 6, wherein steps (b) and (c) can be carried out without intermediate isolation or in a one-pot process.

8. Compound of the general formula (5) or (7) 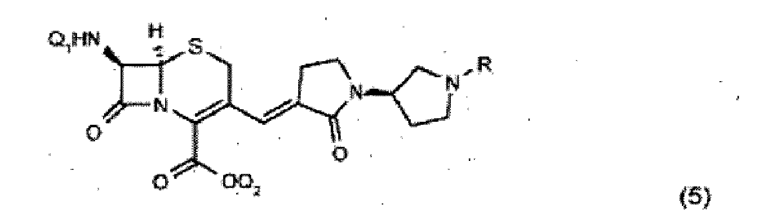 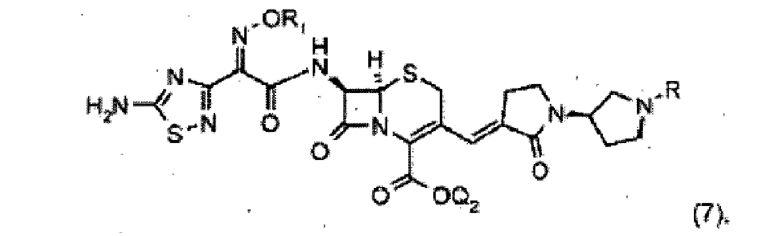 wherein R₁, Q₁, Q₂ and R are as defined according to Claim 1.

9. Compound of the general formula (5) according to Claim 8, 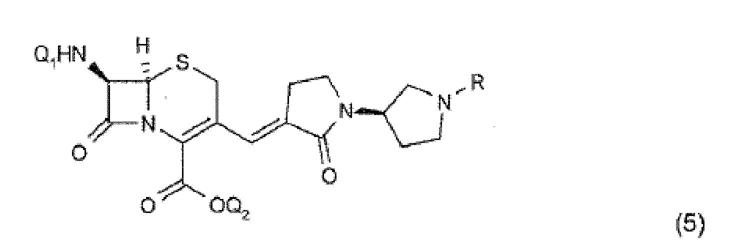 wherein Q₁ represents a trimethylsilyl group, R represents a BOC group and Q₂ represents a silyl group.

10. Use of a compound according to Claim 9 for the production of a compound of the general formula (1)

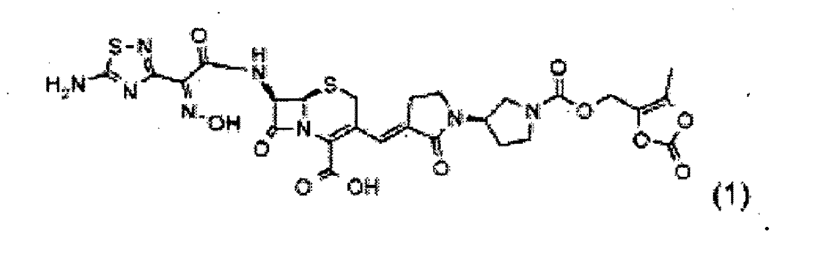

## Revendications

1. Procédé de fabrication d'un composé de formule générale (1) 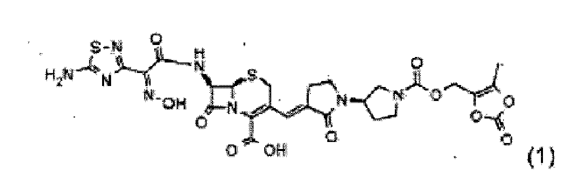 comprenant au moins les étapes (a), (b), (c) et (d) suivantes :
(a) la mise en réaction d'un composé de formule générale (3) 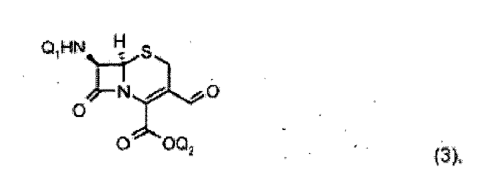 dans laquelle Q₁ et Q₂ représentent indépendamment l'un de l'autre un groupe silyle, ou
dans laquelle Q₁ représente un atome d'hydrogène et Q₂ représente un groupe silyle,
avec un composé de formule générale (4) 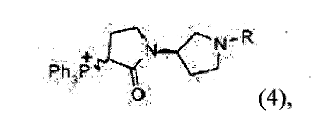 dans laquelle R représente un groupe BOC,
pour former un composé de formule générale (5) 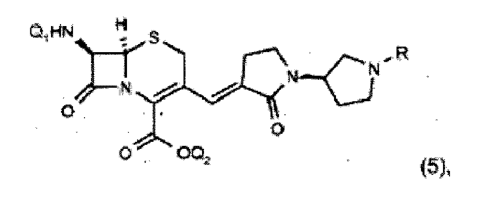 dans laquelle Q₁, Q₂ et R sont tels que définis précédemment ;
(b) la mise en réaction du composé de formule générale (5) avec un composé de formule générale (6) 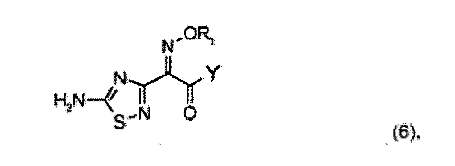 dans laquelle R₁ représente un groupe protecteur hydroxy et Y représente une fonctionnalité activante, éventuellement après élimination du groupe protecteur, si Q₁ représente un groupe silyle,
pour former un composé de formule générale (7) 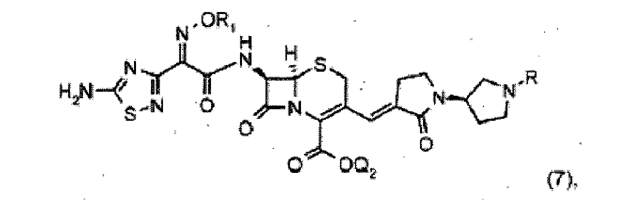 dans laquelle R₁, Q₂ et R sont tels que définis précédemment ;
(c) la mise en réaction du composé de formule générale (7) pour former le composé de formule générale (2), après élimination du groupe protecteur 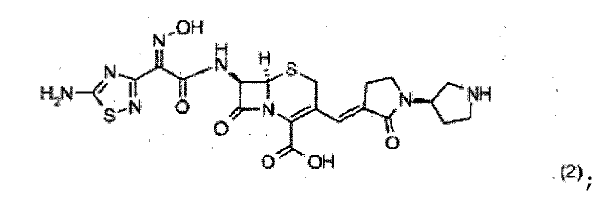 et
(d) la mise en réaction du composé de formule générale (2) pour former un composé de formule générale (1).

2. Procédé de fabrication d'un composé de formule générale (2) 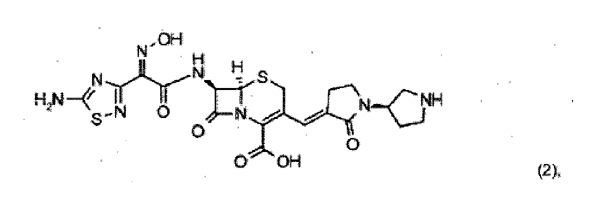 comprenant au moins les étapes (a), (b) et (c) suivantes :
(a) la mise en réaction d'un composé de formule générale (3) 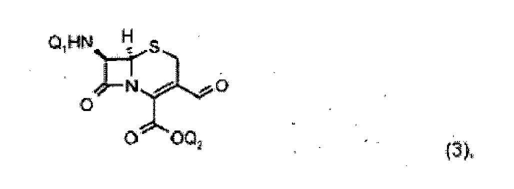 dans laquelle Q₁ et Q₂ représentent indépendamment l'un de l'autre un groupe silyle, ou
dans laquelle Q₁ représente un atome d'hydrogène et Q₂ représente un groupe silyle,
avec un composé de formule générale (4) 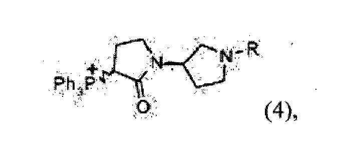 dans laquelle R représente un groupe BOC, pour former un composé de formule générale (5) 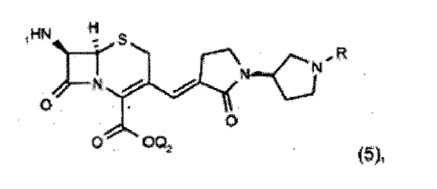 dans laquelle Q₁, Q₂ et R sont tels que définis précédemment ;
(b) la mise en réaction du composé de formule générale (5) avec un composé de formule générale (6) 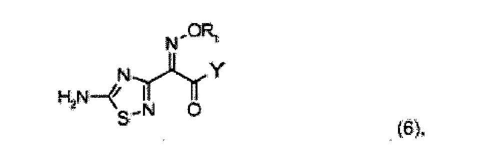 dans laquelle R₁ représente un groupe protecteur hydroxy et Y représente une fonctionnalité activante, éventuellement après élimination du groupe protecteur, si Q₁ représente un groupe silyle,
pour former un composé de formule générale (7) 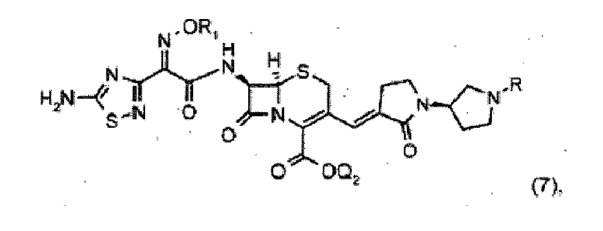 dans laquelle R₁, Q₂ et R sont tels que définis précédemment ;
et
(c) la mise en réaction du composé de formule générale (7) pour former le composé de formule générale (2), après élimination du groupe protecteur.

3. Procédé selon la revendication 1 ou 2, dans lequel l'étape (a) est réalisée en présence d'un agent de silylation et d'un époxyde.

4. Procédé selon la revendication 3, dans lequel l'agent de silylation est l'ASB.

5. Procédé selon la revendication 3 ou 4, dans lequel l'époxyde est l'oxyde de propylène.

6. Procédé selon l'une quelconque des revendications 1 à 5, dans lequel, pour la réaction selon l'étape (b), le composé de formule générale (5) est dissous par silylation ou formation d'un sel, et les groupes protecteurs sont clivés en une étape après la réalisation de l'acylation avec le composé de formule générale (6).

7. Procédé selon l'une quelconque des revendications 1 à 6, dans lequel les étapes (b) et (c) peuvent être réalisées sans isolation intermédiaire ou par un procédé monotope.

8. Composé de formule générale (5) ou (7) 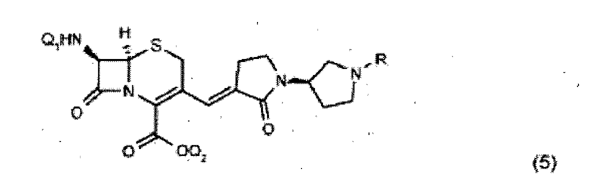 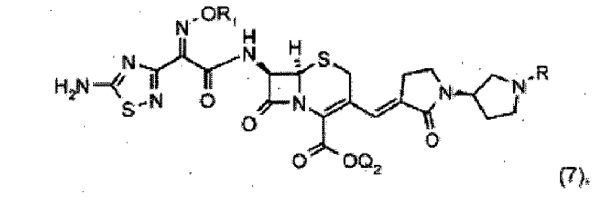 dans lesquelles R₁, Q₁, Q₂ et R sont tels que définis dans la revendication 1.

9. Composé de formule générale (5) selon la revendication 8 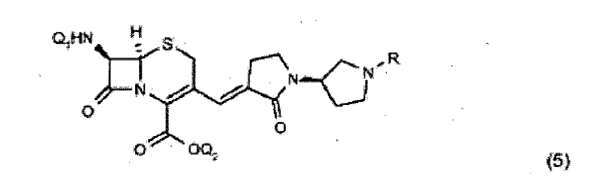 dans laquelle Q₁ représente un groupe triméthylsilyle, R représente un groupe BOC et Q₂ représente un groupe silyle.

10. Utilisation d'un composé selon la revendication 9 pour la fabrication d'un composé de formule générale (1)
